# EUROPÄISCHE PATENTSCHRIFT

(11) **EP 1 192 458 B1**
(45) Veröffentlichungstag und Bekanntmachung des Hinweises auf die Patenterteilung: **16.04.2003**
(21) Anmeldenummer: 99930908.1
(22) Anmeldetag: 07.07.1999
(51) Int. Cl.: G01N 31/22, G01N 33/84, G01N 33/58, G01N 33/533

(54) **OPTISCHER SENSOR FÜR KALIUMIONEN**
OPTICAL SENSOR FOR POTASSIUM IONS
CAPTEUR OPTIQUE POUR IONS DE POTASSIUM

(43) Veröffentlichungstag der Anmeldung: 03.04.2002
(73) Patentinhaber: Andreae, Fritz, 8010 Graz (AT)
(72) Erfinder: ANDREAE, Fritz, A-8010 Graz (AT); URAY, Georg, A-8010 Graz (AT)
(74) Vertreter: Schwarz, Albin, Dr.
(86) Internationale Anmeldenummer: AT9900170
(87) Internationale Veröffentlichungsnummer: WO01004623

(56) Entgegenhaltungen:
- EP-A- 0 861 843
- EP-A- 0 889 324
- WO-A-98/03497
- AT-B- 401 773
- POHL, G. W. ET AL: "Optical and electrical studies on dansyllysine valinomycin in thin lipid membranes" BIOPHYS. STRUCT. MECH. (1976), 2(2), 119-37 , XP000884890

## Beschreibung

Die Erfindung betrifft luminiszenzmarkierte zyklische Peptolide und deren Verwendung zur spezifischen Bestimmung der Konzentration von Kaliumionen in einer Probe. Kalium stellt neben Natrium das wichtigste Kation für die Aufrechterhaltung des zellulären Gleichgewichtes dar. Gerade in der Intensiv- und Notfallsmedizin spielt daher die rasche und zuverlässige Erfassung der Kaliumkonzentration biologischer Systeme in Anwesenheit eines wesentlich höheren Natriumspiegels eine überaus wichtige Rolle. Die Neigung des Kaliums, ebenso wie anderer Alkalimetalle, mit Kronenethern, Kryptanden und Makrolid-Antibiotika Komplexe zu bilden, ist bekannt und läßt sich auch zu deren Analyse ausnützen. Zu den wichtigsten Vertretern der selektiven Kaliumionophore gehört der Naturstoff Valinomycin. Es gehört zur Peptolidklasse der Depsipeptide, welche im Gegensatz zu einfachen Peptiden alternierend aus α-Aminosäuren und α-Hydroxysäuren aufgebaut sind. Valinomycin und auch einige Derivate sind die zur Zeit wirksamsten Komplexbildner. die hochselektiv K⁺ und das biologisch bedeutungslose Rb⁺ selbst in Anwesenheit von weit größeren Mengen Na⁺ und anderen Ionen binden können. Valinomycin ist ein cyclo-Dodeka-Depsipeptid, es stellt daher ein macrozyklisches, 36-gliedriges Ringsystem dar. Es besteht aus 6 α-Aminosäure- und 6 α-Hydroxycarbonsäureeinheiten, nämlich L-Valin, D-Hydroxy-isovaleriansäure, D-Valin und L-Milchsäure in dreifach wiederholter Sequenz. Daher ist es trotz seiner Größe verhältnismäßig einfach aufgebaut und wird mit cyclo(L-Val-D-Hiv-D-Val-L-Lac)₃ in Kurzform beschrieben. Eine Reihe von Varianten mit veränderter Hydroxy- und Aminosäurezusammensetzung ist bekannt. Diese sind ebenfalls mehr oder weniger selektive Kaliumionophore.

In der Literatur sind einige Methoden zur Bestimmung der Kaliumkonzentration mittels des Naturstoffes Valinomycin als Ionophor und einem Fluorophor in einer Matrix beschrieben. Tritt durch Bindung an den Ionophor ein Kaliumion aus der Lösung, bildet sich dadurch eine Potentialdifferenz an der Grenzfläche zwischen Matrix und Lösung aus, die sich in den spektroskopischen Eigenschaften des potentialsensitiven Fluorophors bemerkbar macht.

Es ist aber auch bekannt ( Reinhoudt D. N.: Anal. Chem., 66, 3618, 1994), daß Hersteller derzeitiger Kaliumsensoren auf Valinomycinbasis unter dem Problem des "Auswaschens" einer Komponente aus der den Sensor tragenden Schicht leiden. Dieses Problem ist auch durch eine beschriebene Methode (P. Ross. US 4 973 393 A) der Immobilisierung von Valinomycin nur unzureichend behoben. Denn auch dabei kann es durch "Auswaschen" des Fluorophors zu einer Änderung der molaren Verhältnisse zwischen Fluorophor und Ionophor kommen, was eine exakte und vor allem konstante Messung erschwert. Es wurde dadurch zu verbessern versucht, daß sowohl Ionophor als auch Fluorophor getrennt kovalent in der Matrixschicht eingebettet wurden. Dies macht jedoch eine genaue Kontrolle der jeweils erzielten molaren Verhältnisse der beiden essentiellen Bestandteile notwendig, es führt aber auch leicht zu verschiedenartigen Chargen in der Herstellung. WO 98 03497 A offenbart einen optischen Sensor für Kaliumionen, wobei der lonophor ein fluoreszenzmarkiertes zyklisches Peptolid trägt.

Das beschriebene Problem der ungleichen Molverhältnisse kann dadurch gelöst werden, daß der Ionophor synthetisch hergestellt und dabei kovalent mit dem Label verbunden ist, wodurch das molare Verhältnis der beiden Komponenten naturgemäß konstant bleibt. Der Label kann in einem geeigneten Schritt im Laufe der Synthese an einer Seitenkette eines der Hydroxysäure- bzw. Aminosäurebestandteile an passenden Stelle angebunden werden.

Diese Art der Sensorkonzeption eignet sich bereits sehr gut für die Verwendung in sogenannten Sensorelektroden. Zur fallweise völligen Beseitigung des Problems des Auswaschens und zur größeren Langzeitstabilität wird vorgeschlagen, neben dem Fluorophor gleichzeitig an einer Seitenkette des Ionophors oder des Fluorophors eine zusätzliche funktionelle Gruppe synthetisch einzubauen, die zur kovalenten Bindung an der den Sensor tragenden Schicht geeignet ist.

Zur Verbesserung der Empfindlichkeit und der Anwendungsbreite von Sensorelektroden mit erweiterter Funktion wird erfindungsgemäß der synthetische Einbau eines zweiten Chromophors an geeigneter Stelle des Peptolidgerüsts vorgeschlagen.

Ionophore wie Valinomycin oder andere zyklische Peptolide erfahren bei Komplexierung eines Ions oft eine dramatische Änderung ihrer Konformation. Dadurch kann der bekannte Effekt der molekularen Anziehung von elektronenreichen und elektronenarmen Aromaten (sogenannte "π-Basen" und "π- Säuren") als Meßprinzip genützt werden. Auch stellt der Fluoreszenz Resonanz Energie Transfer (FRET) eine nützliche Interaktion zwischen den elektronischen angeregten Zuständen zweier Farbstoffmoleküle dar. Bei diesem stark distanzabhängigen Effekt wird die Anregungsenergie des Donors direkt ohne Aussendung eines Photons an das Akzeptormolekül übertragen. Derartige Wechselwirkungen werden bei der Enantiomerentrennung ("Pirkle-Phasen") oder bei diversen Enzym-Substrat-Assays seit langem angewandt. Diese Annäherung muß räumlich möglich sein, das heißt, die restlichen vorhandenen Substituenten dürfen nicht den Annäherungsvorgang behindern.

Erfindungsgemäß wird dies dadurch ermöglicht, daß an geeigneten Seitenketten am Ionophor durch kovalente Bindung zwei passende Chromophore als "Labels" befestigt werden. Auch diese Variante sieht eine eventuelle zusätzliche kovalente Fixierung des Gesamtmoleküls an der Matrix vor.

Erfindungsgemäß werden als Ionophore zyklische Peptolide verwendet.

Zur Herstellung dieser Ionophore wird großteils die Synthese an festen Trägern gewählt, wobei man sich der in der Peptidchemie üblichen FMOC- oder t-BOC Synthesestrategie bedient. Im Gegensatz zur herkömmlichen Peptid-synthese werden geschützte "Di-Depsipeptide" als Synthesebausteine verwendet. Die Synthese dieser Bausteine erfolgt mittels konventioneller Synthesemethoden.

Erfindungsgemäß werden für den Aufbau des Ionophors und für die Komplexierung des Kaliums die essentiellen Aminosäurebausteine Glycin (Gly) und Sarcosin (Sar), sowie die optisch aktiven Bausteine Valin (Val), Alanin (Ala), Leucin (Leu) und Isoleucin (Ile) gewählt. Als entsprechende Hydroxykomponenten werden erfindungsgemäß die Enantiomere der Milchsäure (Lac) und Hydroxyisovaleriansäure (Hiv) verwendet.

Zum Aufbau der mehrfach funktionalisierten Ionophore wird eine orthogonale Schutzgruppenstrategie verwendet, die gezielt die Abspaltung einer Schutzgruppe und somit eine selektive Einführung der "Labels" an der funktionellen Seitenketten des Ionophors ermöglicht. Erfindungsgemäß handelt es sich bei diesen funktionellen Bausteinen um Enantiomere der α-Aminosäuren oder α-Hydroxycarbonsäuren mit einer funktionellen Seitenkette, deren Alkylrest zwischen 1 bis 10 Kohlenstoffresten ausmacht und die eine Amino-, Carboxyl-, Hydroxyl-, Thiol-, Sulfonyl- oder eine endständige Olefingruppe, oder ein geeignetes Derivat davon in der Seitenkette besitzen. Bevorzugte Vertreter der Diaminosäurederivate sind Lysin (Lys), Ornitin (Orn) oder Diaminopropionsäure(Dap). Als carboxylhältige Aminosäurederivate eignen sich erfindungsgemäß die Enantiomere der Asparaginsäure (Asp), Glutaminsäure (Glu), oder 2-Amino-Suberinsäure (Asu). Es können aber auch Enantiomere von aromatischen Aminosäurederivaten verwendet werden, wie Nitro-Phenylalanin, Dinitro-Phenylalanin, Amino-Phenylalanin, Jod-Tyrosin oder Tyrosin. Als thiolhältige Aminosäuren sind Cystein (Cys) oder Penicillamin (Pen) geeignet. Alle diese seitenkettenfunktionellen Aminosäuren können durch eine Diazotierungs- und Verkochungsreaktion in die entsprechenden alpha-Hydroxysäuren umgewandelt und eingesetzt werden, sofern sie nicht ohnehin kommerziell erhältlich sind.

Die Erfindung sieht außerdem vor, daß an den funktionellen Seitenketten dieser geänderten Bausteine einerseits die "Labels" kovalent gebunden, anderseits diese auch für eine kovalente Fixierung des Gesamtmoleküls in der Matrix bzw. Sensor tragenden Schicht verwendet werden können. Diese Bindung kann z.B. durch Säureamidbildung einer Carboxylgruppe des Gesamtmoleküls mit einer aminofunktionalisierten PEG-, Acrylatgel- oder Cellulose Matrix erfolgen.

Eine weitere Herstellungsvariante sieht vor, daß bereits "vormarkierte" Synthesebausteine direkt bei der Synthese des Ionophors verwendet werden. Diese Methode der selektiven Einführung von fluoreszenz- oder absorptionsspektroskopisch detektierbaren Gruppen in Peptide und deren Analoga, hier "Labels" genannt, ist bereits in unserem Patent AT 401 773 P ausführlich beschrieben. Diese Methode hat den Vorteil, daß es dabei zu wesentlich besseren Ausbeuten kommt als bei der nachträglichen Einführung der einzelnen Farbstoffe.

Valinomycin selbst weist eine beachtliche Toxizität auf, was in der Verarbeitung als Sensormaterial große Vorsichtsmaßnahmen erfordert. Die Erfindung betrifft daher weiters die Synthese völlig neuer Ionophore auf Basis natürlicher zyklischer Peptolide, in denen sämtliche optisch aktiven Bausteine durch deren optisch aktiven Antipoden ersetzt werden. Als Beispiel wäre das bisher nicht beschriebene Spiegelbildmolekül (Enantiomer) des Valinomycins zu nennen. Anstelle von cyclo(L-Val-D-Hiv-D-Val-L-Lac)₃ wird mit dem ganz gleichen Aufwand cyclo (D-Val-L-Hiv-L-Val-D-Lac)₃ synthetisiert. Erfindungsgemäß hat dies den Vorteil der identen Kompexbildungseigenschaften zum Aufbau von Meßvorrichtungen bei geänderter, meist abgeschwächter oder eliminierter biologischer Wirkung infolge der "unphysiologischen" Chiralität.

### Beispiele

Nomenklatur: Die in den nachfolgenden Valinomycinderivaten verwendete Nomenklatur bezieht sich auf das natürliche Valinomycin. Die Änderung gegenüber dem ursprünglichen Produkt wird durch Angabe der Position und Art des eingesetzten Bausteins, beginnend mit L-Valin in Position 1 indiziert.
(cyclo) ¹L-Val- ²D-Hiv- ³D-Val- ⁴L-Lac- ⁵L-Val- ⁶D-Hiv- ⁷D-Val- ⁸L-Lac- ⁹L-Val- ¹⁰D-Hiv- ¹¹D-Val- ¹²L-Lac

### Beispiel 1:

### FMOC- L-Orn ( 5'(oder 6')-TAMRA)-OH C₄₅H₄₂N₄O₈ [766.85]

### N²-(9-H-Fluoren-⁹yl)-methoxycarbonyl-N⁵-[tetramethylrhodamin-5'-carboxyl-]Ornithin

FMOC-L- Orn. HCl (0.017 mmol) wird in 1.5 ml DMF gelöst und mit einer Lösung von 5'- (oder 6')-TAMRA-NHS (0.16 mmol) in 0.5 ml DMF versetzt. Die über Nacht bei RT gerührte Lösung wird anschließend mit 15 ml Diethylether versetzt und zentrifugiert. Der Rückstand wird erneut mit Ether digeriert und zentrifugiert.

Das so erhaltene dunkelrote Pulver wird mittels "Dry-Flash"- Chromatographie gereinigt.

Die Reinheit und Identität wird per HPLC und Maldi-TOF-Massenspektrometrie überprüft.

Dry-Flash Bedingung: Silica Gel H 15 g ; Laufmittel: CHCl₃:MeOH: Wasser (65:30:5 % v:v)
DC: CHCl₃:MeOH: Wasser (65:35:5 %v:v) Rf.: 0.62
Ausbeute: 85 % d. Theor.

### Beispiel 2:

### ¹L-Orn(t-BOC)-⁷D-Glu(otBu)-Valinomycin C₆₃ H₁₀₅ N₇ O₂₂ [1312,56]

### (cyclo) ¹L-Orn(t-BOC)- ²D-Hiv- ³D-Val- ⁴L-Lac- ⁵L-Val- ⁶D-Hiv- ⁷D-Glu(otBu)- ⁸L-Lac- ⁹L-Val- ¹⁰D-Hiv- ¹¹D-Val- ¹²L-Lac.

Die Synthese erfolgt mittels automatischen Peptid-Synthesizern, wobei anstelle der schrittweise Verknüpfung der einzelnen Bausteine eine Segmentkondensation von geschützten Didepsipeptid-Derivaten erfolgt. Ausgangsmaterial ist ein Trägerharz aus PEG-PS Cogfropolymer welches eine Chlorotrityl-Ankergruppe zur Verknüfung mit dem ersten Didepsipeptid FMOC-D-Val-O-L-Lac besitzt. Die Beladung und Überprüfung der Umsetzungsrate erfolgt nach den in der Peptidchemie typischen Verfahren für Trityl-Trägerharze.

Nach Abspaltung der temporären FMOC-Schutzgruppe erfolgt die schrittweise Verlängerung mit den Didepsipeptidbausteinen : FMOC- L-Val-O-D-Hiv ; FMOC-D-Glu(otBu)-O-L-Lac; FMOC-D-Val-O-L-Lac und FMOC- L-Orn(t-BOC)-O-D-Hiv.

Die Abspaltung der FMOC-Schutzgruppe erfolgt dabei mit 30 %igem Piperidin in DMF.

Die Säureamidbildung bei der Segmentkondensation erfolgt nach der Aktivestermethode unter Verwendung von Diisopropyl-carbodiimid (DIC) und Hydroxybenzotriazol (HOBt).

Die Abspaltung des linearen Valinomycin-Derivates vom Trägerharz wird entweder unter Verwendung von Hexafluoro-isopropanol oder mit einer Spaltlösung bestehend aus Essigsäure : MeOH : Dichlormethan (20: 10: 50 v/v %) innerhalb von 1 Stunde bei RT erzielt. Nach Abtrennung des Trägerharzes wird die Lösung am Rotavapor eingeengt und der entstandene Rückstand durch Lyophilisation von Lösungsmittelresten befreit.
Ausbeute: 85 % Rohausbeute des linearen Valinomycinderivates als weißes Pulver.

Die Reinheit und Identität wird per HPLC und Maldi-TOF-Massenspektrometrie überprüft.
Massenspektrometrie: Matrix Dihydroxybenzoesäure (DHB)
Hauptsignal: 1330,59 MH+ lineares Produkt

Die Reinigung des linearen Produktes erfolgt mittels präparativer Flüssigchromatographie.

Die C-N Zyklisierung erfolgt nach den in der Peptidchemie üblichen Methoden, wobei bevorzugt PyBOP als Kondensationsmittel verwendet wird. Nach Überprüfung der vollständigen Umsetzung mittels HPLC-Analytik und Massenspektrometrie, wird die Lösung mit Wasser versetzt und lyophilisiert. Es bleibt ein amorph kristallines Pulver zurück, welches mittels präparativer HPLC gereinigt wird.
Ausbeute: rd. 35-40 % d. Theor.

### Beispiel 3:

### ¹Orn(5'-FAM)-Valinomycin C₇₅ H₁₀₁ N₇ O₂₄ [1484.66]

### (cyclo) ¹L-Orn(5'-FAM)- ²D-Hiv- ³D-Val- ⁴L-Lac- ⁵L-Val-⁶D-Hiv- ⁷D-Val- ⁸L-Lac- ⁹L-Val- ¹⁰D-Hiv- ¹¹D-Val- ¹²L-Lac.

Die Synthese des cyclo ¹Orn(BOC) - Valinomycin (C₅₉ H₉₉ N₇ O₂₀) erfolgt analog Beispiel 2.

cyclo ¹Orn(BOC) - Valinomycin [1226,47 ] (0.004 mmol) wird in 1 ml Dichlormethan gelöst und mit 1 ml Trifluoressigsäure (TFA) versetzt. Nach 1 Stunde bei RT wird die Lösung am Rotavapor eingeengt, der Rückstand in wäßrigem Acetonitril aufgenommen und lyophilisiert.

Durch HPLC / MS Untersuchung wird die vollständige Abspaltung der O-tBu- und t-BOC-Schutzgruppe überprüft (MH+ 1127.4 m/z).

Der weiße Rückstand wird nun in ca. 500 µl DMF gelöst und nach Zugabe von 0.008 mmol TEA wird 5'-Carboxy-Fluorescin- N-Hydroxysuccinimidylester ( 0.0044 mmol FAM-SE) gelöst in 250 µl DMF zugegeben und die Reaktionslösung ca. 6 - 8 Stunden bei RT geschüttelt.

Die Lösung wird eingeengt und das Produkt mittels präp. HPLC isoliert.
Ausbeute: 31 % d. Theor.

### Beispiel 4:

### ¹Lys(5'-TAMRA)-Valinomycin C₈₀ H₁₁₃ N₉ O₂₂ [1552,82]

### (cyclo) ¹L-Lys(5'-TAMRA)- ²D-Hiv- ³D-Val- ⁴L-Lac- ⁵L-Val- ⁶D-Hiv- ⁷D-Val- ⁸L-Lac- ⁹L-Val- ¹⁰D-Hiv- ¹¹D-Val- ¹²L-Lac.

Die Synthese erfolgt analog wie in Beispiel 2 beschrieben. Anstelle der nachträglichen Einführung des Label an einer Seitenkettenfunktion des Ionophors, wird hier bereits ein "vormarkierter" Synthesebaustein in der Synthese eingesetzt.

In diesem Fall handelt es sich um FMOC-L-Lys(5'-TAMRA)-D-Hiv-OH.
Ausbeute: 45 % d. Theor.

### Beispiel 5:

### ¹D-Lys(5'-TAMRA)-⁷L-Lys(5'-FAM)-Valinomycin C₁₀₂ H₁₂₆ N₁₀ O₂₈ [1940.17]

### (cyclo) ¹L-Lys(5'-FAM)- ²D-Hiv- ³D-Val- ⁴L-Lac- ⁵L-Val- ⁶D-Hiv- ⁷D-Lys(5'- TAMRA)- ⁸L-Lac- ⁹L-Val- ¹⁰D-Hiv- ¹¹D-Val- ¹²L-Lac.

Die Synthese erfolgt analog wie in Beispiel 2 beschrieben. Als Synthesebausteine werden FMOC-D-Lys(t-BOC)-L-Lac und FMOC-L-Lys(Dde)-D-Hiv verwendet. Die Synthese am Harz sowie die Abspaltung erfolgt wie vorhin beschrieben. Die Zyklisierung erfolgt bevorzugterweise mit PyBOP zum zyklischen Valinomycin-Derivat.

Nach präp. HPLC-Reinigung wird eine selektive Abspaltung der einzelnen Seitenkettenschutzgruppen durchgeführt. Dazu wird zuerst mit 2 %iger Hydrazin-Lösung in DMF die Dde - Schutzgruppe vom 'L-Lysin entfernt und anschließend die Spaltlösung durch Lyophilisation entfernt. Der Rückstand wird in DMF aufgenommen und mit Fluorescein als Succinimidylester (SE) umgesetzt. Üblicherweise genügt ein 2.5-facher molarer Überschuß an FAM-SE zur vollständigen Umsetzung. Die Umsetzung wird mittels HPLC überprüft. Nach Entfernen des überschüssigen FAM-SE durch Chromatographie wird das gereinigte Zwischenprodukt lyophilisiert.
Ausbeute an ¹L-Lys(5'-FAM)-⁷D-Lys(BOC)-Valinomycin als Zwischenprodukt : 22 % d. Theor.

Dieses Zwischenprodukt wird nun mit 50 %iger TFA in DCM für rd. 30 min. behandelt, wobei die t-BOC Gruppe von der N^{ε}-Aminofunktion des ⁷Lys entfernt wird. Nach Entfernen der Spaltlösung am Rotavapor wird der Rückstand in wenig DMF aufgenommen und nach Zugabe von einem Äquivalent TEA mit rd. 2.5 Äquivalenten 5'-TAMRA-SE umgesetzt. Nach Überprüfung der Umsetzungsrate mittels HPLC und Massenspektrometrie wird das Produkt mittels präparativer RP-HPLC gereinigt.
Ausbeute: rd. 10-12 % (d. Theor.)

### Beispiel 6:

### ¹Orn(Dabcyl)-⁷D-Glu(EDANS)- Valinomycin C₈₁ H₁₁₄N₁₂ O₂₃S₁ [1655.92]

### (cyclo) ¹L-Orn(DABCYL)- ²D-Hiv- ³D-Val- ⁴L-Lac- ⁵L-Val- ⁶D-Hiv- ⁷D-Glu(EDANS)- ⁸L-Lac- ⁹L-Val- ¹⁰D-Hiv- ¹¹D-Val- ¹²L-Lac.

Die Synthese erfolgt analog wie in Beispiel 4 beschrieben. Als "vormarkierte" Synthesebausteine finden FMOC-L-Orn(Dabcyl)-D-Hiv-OH sowie FMOC-D-Glu(EDANS)-L-Lac-OH in der Synthese Verwendung.
Ausbeute: rd. 38 % (d. Theor.)

### Beispiel 7:

### ¹L-Lys(Bodipy®-FL)-⁵L-Glu-⁹L-Lys(Bodipy®-FL)-Valinomycin C₈₄ H₁₂₀ N₁₂ O₂₂B₂F₄ [1747.55]

### (cyclo) ¹L-Lys(Bodipy®-FL)- ²D-Hiv- ³D-Val- ⁴L-Lac- ⁵L-Glu- ⁶D-Hiv- ⁷D-Val- ⁸L-Lac- ⁹L-Lys(Bodipy®-FL) - ¹⁰D-Hiv- ¹¹D-Val- ¹²L-Lac.

Die Synthese erfolgt wie in Beispiel 2 beschrieben, wobei zusätzlich folgende Synthesebausteinen eingesetzt werden: FMOC-L-Glu(otBu)-D-Hiv, FMOC-L-Lys(t-BOC)-D-Hiv.

Nach Abspaltung , Reinigung und Zyklisierung werden mit TFA alle Schutzgruppen entfernt und Bodipy® als Succinimidylester in Lys in Position 1 und 9 eingeführt. Die zur Immobilisation an der Sensor tragenden Schicht gedachte Carboxylgruppe bleibt dabei unverändert.
Ausbeute: rd. 32 % (d. Theor.)

### Beispiel 8:

### ¹Orn(5'-FAM)-Valinomycin - Kalium-Komplex und Nachweis der selektiven Komplexierung von Kalium- gegenüber Natriumionen C₇₅ H₁₀₁ N₇ O₂₄ K₁ [1523.76]

### Probenvorbereitung

0.5 mg (0.3 µmol) ¹Orn(5'-FAM)-Valinomycin erhalten wie in Beispiel 3 beschrieben, werden in 5 ml Methanol gelöst.

Die massenspektroskopische Analyse mittels Maldi-TOF ergibt als Hauptsignal 1485 m/z als [MH⁺], sowie ein kleineres Signal mit 1523.8 m/z entsprechend [MK⁺]. Entgegen üblichen Maldi-TOF Massenspektren, bei denen immer Natrium- neben Kaliumaddukten nachgewiesen werden können, zeigt sich hier wie bei Valinomycin selbst, als dominantes Nebenprodukt ausschließlich die durch ein

Kalium-Kation komplexierte Form.
- 1000 µl dieser Lösung (0.067 µmol) werden nun mit 15 µl einer 5 mmolaren Natrium-Acetat Lösung inkubiert. Das Massenspektrum zeigt neben 1485.7 m/z als [MH⁺], auch im geringen Ausmaß 1507.65 m/z entsprechend dem Natrium-Komplex [MNa+] sowie 1523.8 m/z entsprechend dem Kalium-Komplex [MK⁺].
- 1000 µl der Lösung (0.067 µmol) werden nun mit 15 µl einer 5 mmolaren Kalium-Acetat Lösung inkubiert. Das Ergebnis des Massenspektrum zeigt ausnahmslos 1523.8 m/z entsprechend dem Kalium-Komplex.
- Wird zu dieser den Kalium-Komplex enthaltenden Lösung nun in steigendem Maße Na-Acetat-Lösung zugegeben, so tritt keinerlei Verdrängung des Kaliums aus dem Komplex durch Natrium auf. Selbst bei 20-fachem Überschuß an Natrium-Ionen konnte kein Auftreten von [MNa⁺] Addukten in der massenspektroskopischen Analyse beobachtet werden.

## Patentansprüche

1. Optischer Sensor für Kaliumionen, wobei der Ionophor ein fluoreszenzmarkiertes zyklisches Peptolid, vorzugsweise ein fluoreszenzmarkiertes Depsipeptid, vorzugsweise ein fluoreszenzmarkierter Abkömmling des Valinomycins oder seines Enantiomers, ist, **dadurch gekennzeichnet, dass** dessen funktionalisierte Seitenketten kovalent gebunden zwei Chromophore tragen.

2. Optischer Sensor nach Anspruch 1, **dadurch gekennzeichnet, dass** der Ionophor eine weitere funktionelle Seitenkette besitzt, die direkt oder nach chemischer Aktivierung zur Immobilisierung an eine den Sensor tragenden Schicht geeignet ist.

3. Optischer Sensor nach Anspruch 1, **dadurch gekennzeichnet, dass** die zur Immobilisierung an einer Matrix geeignete Funktion an einem der Chromophore lokalisiert ist.

4. Optischer Sensor nach einem der Ansprüche 1 bis 3, **dadurch gekennzeichnet, dass** die im Ionophor enthaltenen Aminosäure- und Hydroxycarbonsäurereste Glycin, Sarcosin, Alanin, Valin, Leucin, Isoleucin, Prolin, Hydroxyisovaleriansäure und Milchsäure sind.

5. Optischer Sensor nach einem der Ansprüche 1 bis 4, **dadurch gekennzeichnet, dass** als funktionelle Bausteine zwei bis drei im zyklishen Ionophor enthaltene Reste aus Lysin, Ornithin, Diaminopropionsäure, Asparaginsäure, Glutaminsäure, Aminosuberinsäure, Cystein, Tyrosin und mit einer funktionellen Gruppe am Aromaten substituiertes Phenylalanin oder die entsprechenden daraus durch Austausch der Aminofunktion herstellbaren alpha-Hydroxysäuren eingebaut sind.

6. Optischer Sensor nach einem der Ansprüche 1 bis 5, **dadurch gekennzeichnet, dass** die im Ionophor enthaltenen Aminosäure- und Hydroxycarbonsäurereste jeweils und unabhängig voneinander L-Konfiguration oder D-Konfiguration besitzen.

7. Optischer Sensor nach einem der Anspruche 1 bis 6, **dadurch gekennzeichnet, dass** die zur Immobilisierung an einer Matrix geeignete Seitenkette des Ionophors oder des Chromophors eine Carboxyl-, Amino-, Thiol-, Isocyanat-, Thiocyanat- oder Sulfonylgruppe tragen, die direkt oder nach chemischer Aktivierung zur Immobilisierung an eine den Sensor tragenden Schicht geeignet sind.

8. Optischer Sensor nach einem der Ansprüche 1 bis 7, **dadurch gekennzeichnet, dass** die kovalent am Ionophor befestigten Chromophore aus der Gruppe bestehend aus TAMRA, Oregon-Green™, FAM, EDANS, Bodipy-FL®, Dabcyl, Dabsyl, Texas-Red® oder Cyanin-Farbstoffen ausgewählt sind.

9. Optischer Sensor nach einem der Ansprüche 1 bis 8, **dadurch gekennzeichnet, dass** sich durch die Bindung des zu messenden Ions mindestens eine optische Eigenschaft mindestens eines Chromophors ändert.

## Claims

1. An optical sensor for potassium ions, wherein the ionophore is a fluorescence-labelled cyclic peptolide, preferably a fluorescence-labelled depsipeptide, preferably a fluorescence-labelled derivative of the valinomycin or of its enantiomer, **characterized in that** its functionalized side-chains have covalently bound thereto two chromophores.

2. An optical sensor according to claim 1, **characterized in that** the ionophore has a further functional side-chain suitable for being immobilized onto a layer carrying the sensor, either directly or after being chemically activated.

3. An optical sensor according to claim 1, **characterized in that** the function suitable for immobilization on a matrix is located on one of the chromophores.

4. An optical sensor according to any one of claims 1 to 3, **characterized in that** the amino acid and hydroxycarboxylic acid residues included in the ionophore are glycine, sarcosine, alanine, valine, leucine, isoleucine, proline, hydroxyisovaleric acid and lactic acid.

5. An optical sensor according to any one of claims 1 to 4, **characterized in that** two to three residues of lysine, ornithine, diaminopropionic acid, aspartic acid, glutamic acid, aminosuberic acid, cysteine, tyrosine and phenylalanine substituted at the aromatic compound with a functional group or the respective alpha-hydroxy acids preparable therefrom by changing the amino function, which are included in the cyclic ionophore, are incorporated as functional components.

6. An optical sensor according to any one of claims 1 to 5, **characterized in that** the amino acid and hydroxycarboxylic acid residues included in the ionophore each and independently of each other possess an L-configuration or a D-configuration.

7. An optical sensor according to any one of claims 1 to 6, **characterized in that** the side-chain of the ionophore or of the chromophore, which side-chain is suitable for immobilization on a matrix, has a carboxyl, amino, thiol, isocyanate, thiocyanate or sulfonyl group suitable for being immobilized onto a layer carrying the sensor, either directly or after being chemically activated.

8. An optical sensor according to any one of claims 1 to 7, **characterized in that** the chromophores covalently attached to the ionophore are selected from the group consisting of TAMRA, Oregon-Green™, FAM, EDANS, Bodipy-FL®, Dabcyl, Dabsyl, Texas-Red® or cyanine dyes.

9. An optical sensor according to any one of claims 1 to 8, **characterized in that** at least one optical property of at least one chromophore changes because of the bonding of the ion to be measured.

## Revendications

1. Capteur optique pour les ions potassium, dans lequel l'ionophore est un peptolide cyclique à marqueur fluorescent, de préférence un depsipeptide à marqueur fluorescent, de préférence un dérivé de la valinomycine ou de son énantiomère à marqueur fluorescent, **caractérisé en ce que** ses chaînes latérales fonctionnalisées portent deux chromophoxes liés de manière covalente.

2. Capteur optique selon la revendication 1, **caractérisé en ce que** l'ionophore possède une chaîne latérale fonctionnelle supplémentaire qui, directement ou après activation chimique, convient à l'immobilisation sur une couche portant le capteur.

3. Capteur optique selon la revendication 1, **caractérisé en ce que** la fonction convenant à l'immobilisation sur une matrice est localisée sur un des chromophores.

4. Capteur optique selon l'une quelconque des revendications 1 à 3, **caractérisé en ce que** les résidus d'acide aminé et d'acide hydroxycarboxylique contenus dans l'ionophore sont la glycine, la sarcosine, l'alanine, la valine, la leucine, l'isoleucine, la proline, l'acide hydroxyisovalérique, et l'acide lactique.

5. Capteur optique selon l'une quelconque des revendications 1 à 4, **caractérisé en ce que** deux à trois résidus contenus dans l'ionophore cyclique, constitués de lysine, d'ornithine, d'acide diaminopropionique, d'acide aspartique, d'acide glutamique, d'acide aminosubérique, de cystéine, de tyrosine et de phénylalanine substituée par un groupe fonctionnel sur le groupe aromatique, ou les alpha-hydroxyacides que l'on peut préparer de manière appropriée à partir de ceux-ci par échange de la fonction amino, sont incorporés en tant que composants fonctionnels.

6. Capteur optique selon l'une quelconque des revendications 1 à 5, **caractérisé en ce que** les résidus d'acide aminé et d'acide hydroxycarboxylique contenus dans l'ionophore possèdent chacun et indépendamment l'un de l'autre une configuration L ou une configuration D.

7. Capteur optique selon l'une quelconque des revendications 1 à 6, **caractérisé en ce que** la chaîne latérale de l'ionophore ou du chromophore, convenant à l'immobilisation sur une matrice, porte un groupe carboxyle, amino, thiol, isocyanate, thiocyanate ou sulfonyle, qui, directement ou après activation chimique, conviennent à l'immobilisation sur une couche portant le capteur.

8. Capteur optique selon l'une quelconque des revendications 1 à 7, **caractérisé en ce que** les chromophores fixés de manière covalente à l'ionophore sont choisis dans le groupe formé par TAMRA, Oregon-Green™, FAM, EDANS, Bodipy-FL®, Dabcyl, Dabsyl, Texas-Red®, ou les colorants à base de cyanine.

9. Capteur optique selon l'une quelconque des revendications 1 à 8, **caractérisé en ce qu'**au moins une propriété optique d'un chromophore se modifie par la fixation de l'ion à mesurer.
